# EUROPÄISCHE PATENTSCHRIFT

(11) **EP 2 234 557 B1**
(45) Veröffentlichungstag und Bekanntmachung des Hinweises auf die Patenterteilung: **04.12.2013**
(21) Anmeldenummer: 09705663.4
(22) Anmeldetag: 21.01.2009
(51) Int. Cl.: A61B 18/14, A61M 1/00

(54) **BIPOLARES KOAGULATIONSINSTRUMENT**
BIPOLAR COAGULATION INSTRUMENT
INSTRUMENT DE COAGULATION BIPOLAIRE

(30) Priorität: 31.01.2008 DE 102008006880
(43) Veröffentlichungstag der Anmeldung: 06.10.2010
(73) Patentinhaber: Karl Storz GmbH & Co. KG, 78532 Tuttlingen (DE)
(72) Erfinder: BACHER, Uwe, 78532 Tuttlingen (DE); HERMLE, Rainer, 78559 Gosheim (DE)
(74) Vertreter: Hofmeister, Frank
(86) Internationale Anmeldenummer: PCT/EP2009/000344
(87) Internationale Veröffentlichungsnummer: WO 2009/095172

(56) Entgegenhaltungen:
- WO-A-2006/092563
- WO-A-2006/102124
- WO-A1-93/09839
- DE-A1- 4 138 115
- DE-A1- 19 541 566
- DE-A1-102004 033 595
- DE-C1- 4 439 553
- DE-U1- 20 117 907
- GB-A- 2 406 057

## Beschreibung

Die Erfindung betrifft ein bipolares Koagulationsinstrument mit einem als Saugund/oder Spülrohr ausgebildeten hohlen Schaft sowie mit zwei gegeneinander elektrisch isolierten Elektroden, die das distale Ende des Schaftes überragen, wobei der hohle Schaft aus zwei koaxial aufeinander angeordneten, zumindest teilweise elektrisch leitenden Rohren besteht, die gegeneinander und nach außen elektrisch isoliert sind, wobei die Rohre die Elektroden bilden.

Ein gattungsgemäßes bipolares Koagulationsinstrument ist beispielsweise aus der GB 2 406 057 A bekannt. Bei diesem bekannten medizinischen Instrument besteht der Schaft aus zwei koaxial zueinander angeordneten Rohren, die jeweils aus einem elektrisch leitfähigen Material bestehen und die durch Isolierungen gegeneinander und nach außen isoliert sind. Diese beiden elektrisch leitfähigen Rohre bilden die Elektroden eines Koagulationsinstruments.

Zum Schneiden des zu entfernenden Gewebes ist angrenzend an den distalseitigen Verschluss des Außenrohres eine maulartige Öffnung in der Mantelfläche des Außenrohres ausgebildet. Eine entsprechende maulartige Öffnung findet sich auch in der Mantelfläche des koaxial im Inneren des Außenrohres gelagerten Innenrohr. Bei dieser Ausgestaltung wirken die gesamten Umfangsränder der maulartigen Öffnungen im Innen- und Außenrohr als aktive Schneidelektroden.

Weiterhin ist dieses bekannte Koagulationsinstrument über einen Anschluss an einen externen Spülflüssigkeitsbehälter sowie über einen weiteren Anschluss an eine externe Saugvorrichtung anschließbar.

Ein weiteres bipolares Koagulationsinstrument ist beispielsweise aus der DE 44 39 553 C1 bekannt. Derartige bipolare Koagulationsinstrumente werden beispielsweise in der Nasen-Nebenhöhlen-Chirurgie verwendet, um zusätzlich zur Koagulationsbehandlung, bei der über die mit Strom beaufschlagten Elektroden Gefäße zu verschließen. Die Kombination eines bipolares Koagulationsinstruments mit einem Saug- und/oder Spülinstrument ist vorteilhaft, da das bei Operationen aus geöffneten Gefäßen austretende Blut die Sicht auf das Operationsgebiet behindert. In diesen Fällen ist es erforderlich, das Operationsgebiet zunächst freizuspülen und/oder freizusaugen, bevor die Gefäße durch Koagulation wieder verschlossen werden können.

Zur Regulierung des Saugdrucks über den Sauganschluss ist im Bereich des Sauganschlusses in der Handhabe eine mit einem Finger verschließbare Öffnung ausgebildet.

Ein weiteres bipolares Instrument mit regulierbarer Saugkraft ist aus der WO 93/09839 A1 bekannt.

Nachteilig bei den bekannten bipolaren Koagulationsinstrumenten ist, dass aufgrund der Stromzuführungskabel hin zu den am distalen Ende des Schaftes angeordneten Elektroden der zum Saugen und/oder Spülen zur Verfügung stehende freie Querschnitt des hohlen Schaftes deutlich verringert ist, so dass die Saug/Spülleistung der bekannten Instrumente bei starken Blutungen nur ungenügend ist. Darüber hinaus weisen die bekannten Instrumente, insbesondere im Bereich der Elektroden, einen komplexen, aus vielen einzelnen Kleinbauteilen bestehenden Aufbau auf, der die Montage erschwert und verteuert.

Davon ausgehend liegt der Erfindung die **Aufgabe** zugrunde, ein bipolares Koagulationsinstrument der eingangs genannten Art zu schaffen, das bei einfachem Aufbau eine effektive Saug-/Spülleistung gewährleistet.

Die **Lösung** dieser Aufgabenstellung ist erfindungsgemäß dadurch gekennzeichnet, dass im Schaft eine Drosselöffnung ausgebildet ist, wobei im Bereich der Drosselöffnung im äußeren Rohr eine die Drosselöffnung allseitig mit Abstand umgebende Aussparung ausgebildet ist.

Durch die Ausbildung der Elektroden als koaxial zueinander angeordnete elektrisch leitende Rohre ist es möglich, den gesamtem freien Querschnitt des hohlen

Schaftes als Saug- und/oder Spülkanal zur Verfügung zu stellen, da es keiner Verkabelung im Inneren des Schaftes mehr bedarf. Die Isolation der Rohre gegeneinander und gegenüber der Umgebung erfolgt über die isolierenden Schichten, die auf dem inneren Rohr und dem äußeren Rohr angeordnet sind.

Um die Saugleistung des über den hohlen Schaft abgesaugten Volumenstroms einfach und schnell regulieren zu können, ist erfindungsgemäß im Schaft eine Drosselöffnung ausgebildet, die der Bediener des erfindungsgemäßen Koagulationsinstruments mit einem Finger öffnen und schließen kann. Die Drosselöffnung ist dabei vorteilhafterweise so dimensioniert, dass bei vollständig geöffneter Drosselöffnung die Saugleistung im wesentlichen zum Erliegen kommt.

Da bei dem erfindungsgemäßen Koagulationsinstrument beide den Schaft bildenden Rohre als elektrisch leitende Elektroden ausgebildet sind, ist im Bereich der Drosselöffnung im äußeren Rohr eine die Drosselöffnung allseitig mit Abstand umgebende Aussparung ausgebildet ist, um einen Kurzschluss zwischen den beiden Elektroden im Bereich der Drosselöffnung zu verhindern.

Gemäß einer praktischen Ausführungsform der Erfindung wird vorgeschlagen, dass die Rohre über die Rohre koaxial umgebende Schichten gegeneinander und nach außen elektrisch isoliert sind, wobei zwei Rohre und die zwei isolierenden Schichten vorteilhafterweise formschlüssig aneinander anliegen, um eine besonders kompakte Bauweise des Schaftes zu ermöglichen.

Zur Erzielung einer im Wesentlichen vollflächigen und formschlüssigen Anlage der isolierenden Schichten an den jeweiligen Rohren wird erfindungsgemäß vorgeschlagen, dass die isolierenden Schichten als auf das innere Rohr und das äußere Rohr aufgebrachte Schläuche, insbesondere Schrumpfschläuche, ausgebildet sind.

Gemäß einer alternativen Ausführungsform der Erfindung wird vorgeschlagen, dass isolierenden Schichten als auf das innere Rohr und das äußere Rohr aufgebrachte Beschichtungen aus einem Kunststoff- oder Keramikmaterial ausgebildet sind.

Weiterhin wird mit der Erfindung vorgeschlagen, dass zur Ausbildung der eigentlichen Arbeitselektroden an den distalen Enden des Innenrohres und des Außenrohres fingerartig vorspringende Rohrabschnitte ausgebildet sind, die das distale Ende des Schaftes überragen, wobei diese fingerartig vorspringenden Rohrabschnitte, vorteilhafterweise einander gegenüberliegend, am distalen Ende des Schaftes angeordnet sind.

Um im Bereich der distalseitigen freien Enden der Rohre eine formschlüssige Anlage und spaltfreie Abdichtung der isolierenden Schichten an den Rohren zu gewährleisten, wird mit der Erfindung weiterhin vorgeschlagen, dass an den freien Enden der Rohre im Übergang zu den vorstehenden Elektrodenspitzen den Außendurchmesser der Rohre reduzierende, nach schräg innen verlaufende Anschliffflächen ausgebildet sind.

Eine einfache Handhabung der Drosselöffnung ist erfindungsgemäß dadurch erreichbar, dass die Drosselöffnung im Bereich einer am proximalen Ende des Schaftes angeordneten Handhabe ausgebildet ist, so dass der Bediener das Instrument mit nur einer Hand halten und gleichzeitig auch die Drosselöffnung bedienen kann.

Um ein einwandfreies Verschließen der Drosselöffnung mittels eines Fingers des Instrumentenbenutzers zu gewährleisten, wird mit der Erfindung weiterhin vorgeschlagen, dass auch in der auf dem äußeren Rohr angeordneten isolierenden Schicht eine die Drosselöffnung allseitig mit Abstand umgebende Aussparung ausgebildet ist.

Weitere Merkmale und Vorteile der Erfindung ergeben sich anhand der zugehörigen Zeichnung, in der ein Ausführungsbeispiel eines erfindungsgemäßen bipolaren Koagulationsinstruments nur beispielhaft dargestellt ist, ohne die Erfindung auf dieses Ausführungsbeispiel zu beschränken. In der Zeichnung zeigt:
- Fig. 1: eine perspektivische Seitenansicht eines erfindungsgemäßen bipolaren Koagulationsinstruments;
- Fig. 2: eine Explosionszeichnung des Instruments gemäß Fig. 1;
- Fig. 3: eine teilweise aufgeschnittene vergrößerte Darstellung des Details III gemäß Fig. 1
- Fig. 4: einen Schnitt entlang der Linie IV-IV gemäß Fig. 1;
- Fig. 5: eine Draufsicht auf das proximale Ende des Instruments gemäß Fig. 1 und
- Fig. 6: eine teilweise aufgeschnittene vergrößerte Darstellung des Details VI gemäß Fig. 5, jedoch ohne Handhabe.

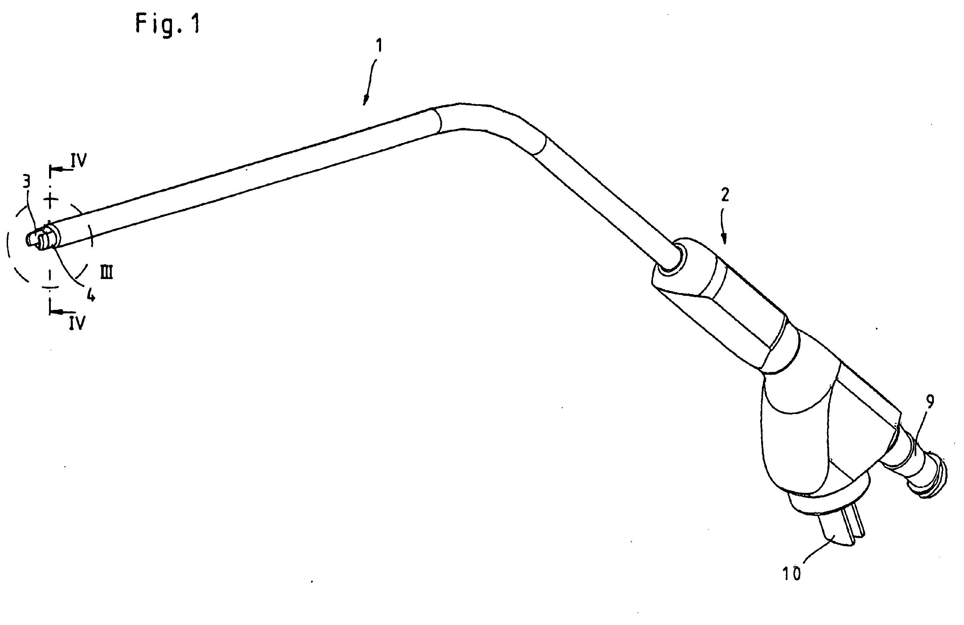

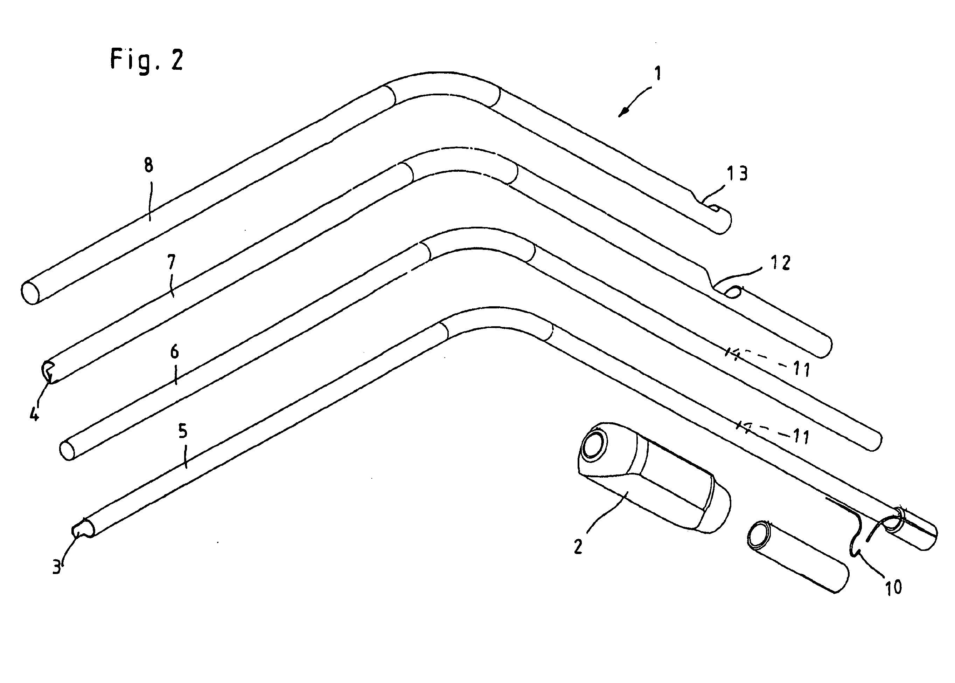

Das insbesondere in den Abbildungen Fig. 1 und 2 dargestellte medizinische bipolaren Koagulationsinstrument besteht im Wesentlichen aus einem hohlen, als Saug-/Spülkanal ausgebildeten Schaft 1, einer am proximalen Ende des Schaftes 1 angeordneten Handhabe 2, sowie zwei den hohlen Schaft 1 distalseitig überragenden Elektrodenspitzen 3 und 4.

Wie insbesondere aus Fig. 2 ersichtlich, ist der hohle Schaft 1 mehrlagig ausgebildet, bestehend aus einem inneren Rohr 5, einer das innere Rohr 5 koaxial umgebenden elektrisch isolierenden Schicht 6, einem das innere Rohr 5 sowie die isolierende Schicht 6 koaxial umgebenden äußeren Rohr 7 sowie einer das äußere Rohr 7 koaxial umgebenden elektrisch isolierenden Schicht 8. Bei der dargestellten Ausführungsform sind die isolierenden Schichten 6 und 8, die einerseits die Rohre 5 und 7 gegeneinander elektrisch isolieren und andererseits den Schaft 1 nach außen elektrisch isolieren, als die Rohre 5 und 7 formschlüssig umgebende Schrumpfschläuche ausgebildet.

Selbstverständlich ist es auch möglich, die elektrische Isolierung der Rohre 5 und 7 gegeneinander und zur Umgebung hin auch mittels anderer elektrisch isolierender Schichten, wie beispielsweise das Beschichten der Rohre 5 und 7 mit elektrisch isolierenden Schichten aus einem Kunststoff- oder Keramikmaterial, zu bewerkstelligen.

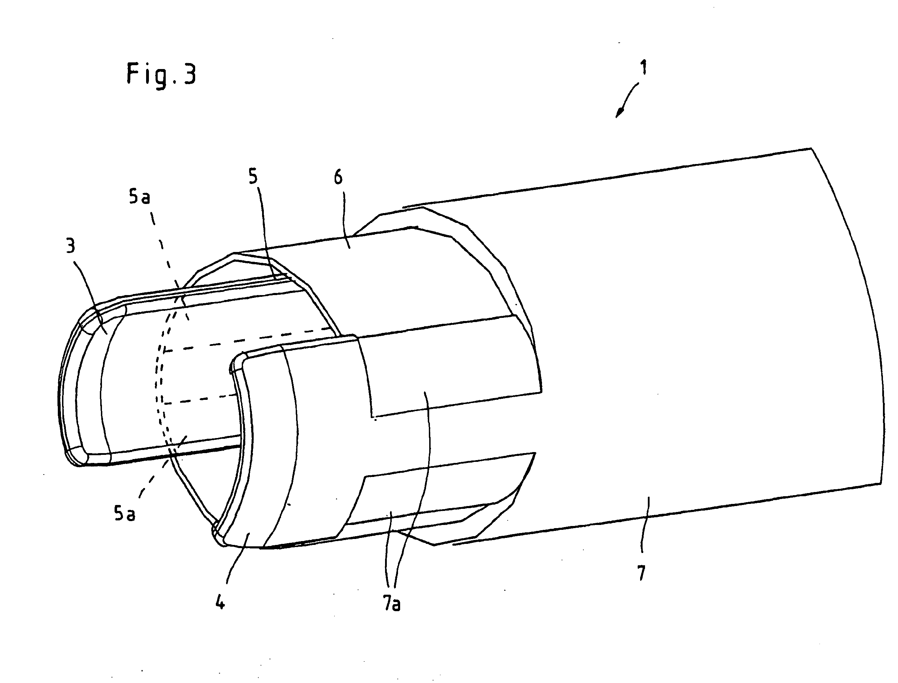

Die Rohre 5 und 7 ihrerseits sind zumindest teilweise elektrisch leitend ausgebildet, so dass die Rohre 5 und 7 gleichzeitig die Elektroden des bipolaren Koagulationsinstruments bilden. Zur Ausbildung der eigentlichen Elektrodenspitzen 3 und 4 sind an den distalen Enden des Innenrohres 5 und des Außenrohres 7 fingerartig vorspringende Rohrabschnitte ausgebildet, die das distale Ende des Schaftes 1 überragen, wie dies in Fig. 1 und 3 dargestellt ist. Diese fingerartig vorspringenden Elektrodenspitzen 3 und 4 sind einander gegenüberliegend am distalen Ende des Schaftes 1 angeordnet.

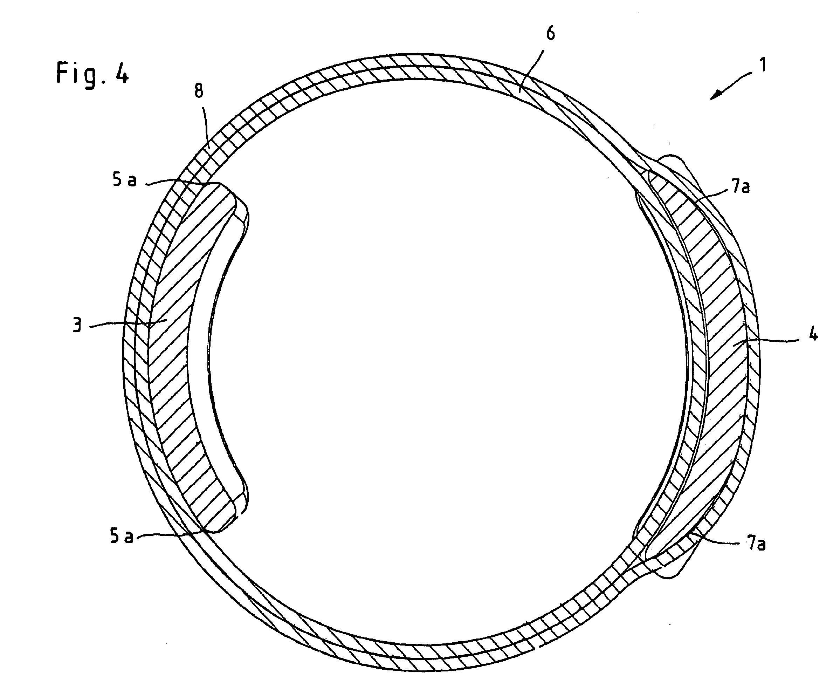

Der mehrlagige Aufbau des hohlen Schaftes 1 ist neben der Explosionszeichnung gemäß Fig. 2 insbesondere den Abbildungen Fig. 3 und 4 zu entnehmen, die das die Elektrodenspitzen 3 und 4 aufweisende distale Ende des Schaftes 1 zeigen. In Fig. 3 wurde zur besseren Verdeutlichung der Ausbildung der Elektrodenspitzen 3 und 4 als distale Verlängerung der Rohre 5 und 7 die äußere isolierende Schicht 8 weggelassen.

Den Abbildung Fig. 3 und 4 ist weiterhin zu entnehmen, wie die Rohre 5 und 7 im Bereich des Übergangs zu den Elektrodenspitzen 3 und 4 geometrisch ausgebildet sind. Um im Bereich der distalseitigen freien Enden der Rohre 5 und 7 eine formschlüssige Anlage und spaltfreie Abdichtung der isolierenden Schichten 6 und 8 an den Rohren 5 und 7 zu gewährleisten, sind an den freien Enden der Rohre 5 und 7 im Übergang zu den vorstehenden Elektrodenspitzen 3 und 4 den Außendurchmesser der Rohre 5 und 7 reduzierende, nach schräg innen verlaufende Anschliffflächen 5a und 7a ausgebildet.

Die Ausbildung der den hohlen Schaft 1 bildenden Rohre 5 und 7 als Elektroden des bipolaren Koagulationsinstruments hat den Vorteil, dass der gesamte freie Querschnitt des hohlen Schaftes 1 als Saug- und/oder Spülkanal zur Verfügung steht und es keiner Verkabelung im Inneren des Schaftes 1 bedarf.

Zur Ausbildung des hohlen Schaftes 1 als Saug- und/oder Spülkanal weist die Handhabe 2 an ihrem proximalen Ende einen Saug- und/oder Spülanschluss 9 zur Ankopplung einer externen Saug- und/oder Spülleitung auf.

Weiterhin weist die Handhabe 2 einen Stromanschluss 10 auf, über den die als Elektroden ausgebildeten Rohre 5 und 7 mit Strom beaufschlagbar sind.

Um dem Operateur zu ermöglichen, die Saugleistung über den als Saug- und/oder Spülkanal ausgebildeten hohlen Schaft 1 einfach und schnell regulieren zu können, weist der hohle Schaft 1 im Bereich der Handhabe 2 eine Drosselöffnung 11 auf, die das Innere des Saug- und/oder Spülkanals mit der Umgebungsluft verbindet. Bei der dargestellten Ausführungsform ist die Drosselöffnung 11 als im wesentlichen radial verlaufende Bohrung ausgebildet. Selbstverständlich sind auch anders geformte Drosselöffnungen 11, wie beispielsweise oval, rechteckig oder dergleichen ausgebildete Öffnungen, darstellbar.

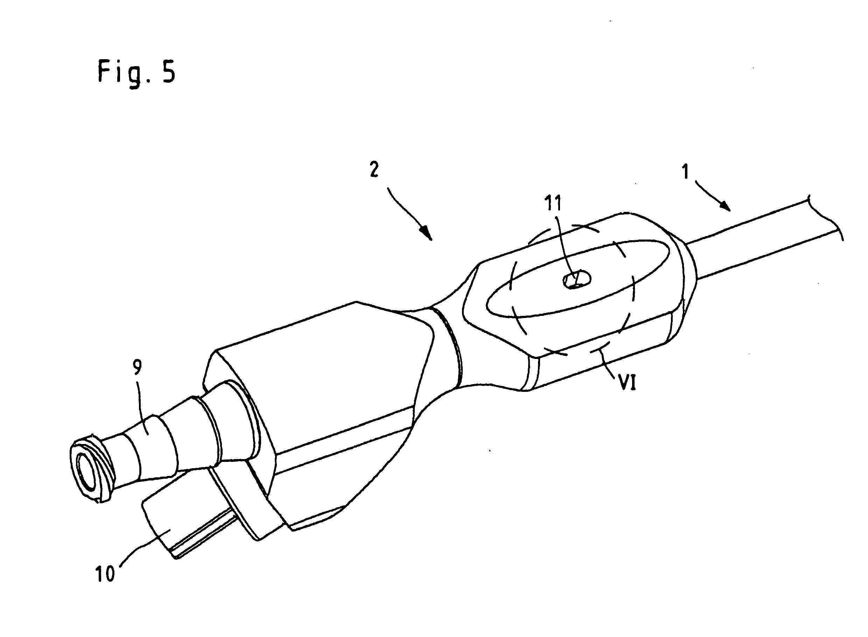

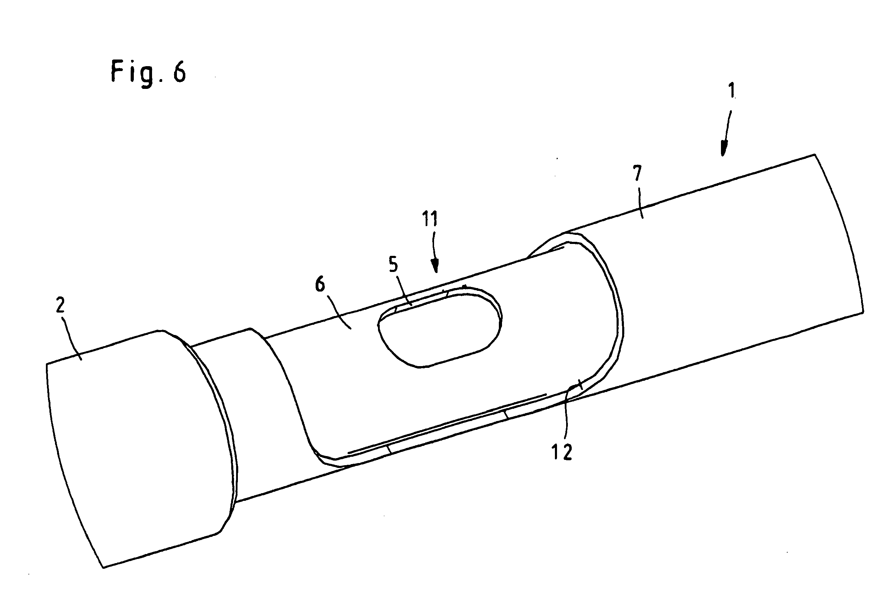

Die insbesondere den Abbildungen Fig. 5 und 6 zu entnehmende Drosselöffnung 11 ist so ausgebildet und an der Handhabe platziert, dass der Operateur die Drosselöffnung 11 mit einem Finger öffnen und wieder verschließen kann, beispielsweise mit einem Finger der Hand, mit der er auch die Handhabe 2 zum Führen des Instruments hält.

Die Drosselöffnung 11 ist dabei vorteilhafterweise so dimensioniert, dass bei vollständig geöffneter Drosselöffnung 11 die Saugleistung im wesentlichen zum Erliegen kommt, da über die geöffnete Drosselöffnung 11 so viel Umgebungsluft angesaugt wird, dass die Saugleistung nicht bis zum distalen Ende des Schaftes 1 gelangt. Je weiter der Operateur die Drosselöffnung 11 mit seinem Finger verschließt desto stärker wird die Saugleistung im Inneren des hohlen Schaftes 1 und somit die Absaugleistung aus dem Operationsgebiet über das distale Ende des Schaftes 1.

Um zu Verhindern, dass es im Bereich der Drosselöffnung 11 durch Flüssigkeit oder Überbrückung durch den Finger des Operateurs zu einem Kurzschluss zwischen den als Elektroden aufgebauten Rohren 5 und 7 kommt, ist im Bereich der Drosselöffnung 11 im äußeren Rohr 7 eine die Drosselöffnung 11 allseitig mit Abstand umgebende Aussparung 12, beispielsweise in Form einer Freifräsung, ausgebildet, wie dies der Abbildung Fig. 6 zu entnehmen ist, bei der aus Gründen der besseren Übersichtlichkeit die äußere isolierende Schicht 8 weggelassen wurde.

Wie aus Fig. 2 ersichtlich, weist auch die äußere isolierende Schicht 8 im Bereich der Drosselöffnung 11 eine die Drosselöffnung 11 allseitig mit Abstand umgebende Aussparung 13 auf, um ein einwandfreies Verschließen der Drosselöffnung 11 mittels eines Fingers des Operateurs zu gewährleisten.

Ein wie voran stehend beschriebenes bipolares Koagulationsinstrument zeichnet sich insbesondere dadurch aus, dass es einerseits aus nur wenigen Bauteilen bestehend einfach aufgebaut ist und somit kostengünstig montierbar ist und andererseits, aufgrund der Ausbildung der den hohlen Schaft 1 bildenden Rohre 5 und 7 als Elektroden des bipolaren Koagulationsinstruments, der gesamte freie Querschnitt des hohlen Schaftes 1 als Saug- und/oder Spülkanal zur Verfügung steht.

Weiterhin erlaubt die Ausbildung der Drosselöffnung 11 ein einfaches, schnelles und effektives Regulieren der Saugleistung im Inneren des als Saug- und/oder Spülkanal ausgebildeten hohlen Schaftes 1.

### Bezugszeichenliste

- 1: Schaft
- 2: Handhabe
- 3: Elektrodenspitze/Rohrabschnitt
- 4: Elektrodenspitze/Rohrabschnitt
- 5: inneres Rohr
- 5a: Anschlifffläche
- 6: isolierende Schicht
- 7: äußeres Rohr
- 7a: Anschlifffläche
- 8: isolierende Schicht
- 9: Saug- und/oder Spülanschluss
- 10: Stromanschluss
- 11: Drosselöffnung
- 12: Aussparung
- 13: Aussparung

## Patentansprüche

1. Bipolares Koagulationsinstrument mit einem als Saug- und/oder Spülrohr ausgebildeten hohlen Schaft (1) sowie mit zwei gegeneinander elektrisch isolierten Elektroden (3, 4), die das distale Ende des Schaftes (1) überragen, wobei der hohle Schaft (1) aus zwei koaxial aufeinander angeordneten, zumindest teilweise elektrisch leitenden Rohren (5, 7) besteht, die gegeneinander und nach außen elektrisch isoliert sind, wobei die Rohre (5, 7) die Elektroden (3, 4) bilden,
**dadurch gekennzeichnet,**
**dass** im Schaft (1) eine Drosselöffnung (11) ausgebildet ist, wobei im Bereich der Drosselöffnung (11) im äußeren Rohr (7) eine die Drosselöffnung (11) allseitig mit Abstand umgebende Aussparung (12) ausgebildet ist.

2. Bipolares Koagulationsinstrument nach Anspruch 1, **dadurch gekennzeichnet, dass** die Rohre (5, 7) über zwei die Rohre (5, 7) koaxial umgebende Schichten (6, 8) gegeneinander und nach außen elektrisch isoliert sind.

3. Bipolares Koagulationsinstrument nach Anspruch 2, **dadurch gekennzeichnet, dass** die zwei Rohre (5, 7) und die zwei isolierenden Schichten (6, 8) formschlüssig aneinander anliegen.

4. Bipolares Koagulationsinstrument nach Anspruch 2 oder 3, **dadurch gekennzeichnet, dass** die isolierenden Schichten (6, 8) als auf das innere Rohr (5) und das äußere Rohr (7) aufgebrachte Schläuche, insbesondere Schrumpfschläuche, ausgebildet sind.

5. Bipolares Koagulationsinstrument nach Anspruch 2 oder 3, **dadurch gekennzeichnet, dass** die isolierenden Schichten (6, 8) als auf das innere Rohr (5) und das äußere Rohr (7) aufgebrachte Beschichtungen aus einem Kunststoffoder Keramikmaterial ausgebildet sind.

6. Bipolares Koagulationsinstrument nach einem der Ansprüche 1 bis 5, **dadurch gekennzeichnet, dass** an den distalen Enden des Innenrohres (5) und des Außenrohres (7) als Elektrodenspitzen fingerartig vorspringende Rohrabschnitte (3, 4) ausgebildet sind, die das distale Ende des Schaftes (1) überragen.

7. Bipolares Koagulationsinstrument nach Anspruch 6, **dadurch gekennzeichnet, dass** die fingerartig vorspringenden Rohrabschnitte (3, 4) einander gegenüberliegend am distalen Ende des Schaftes (1) angeordnet sind.

8. Bipolares Koagulationsinstrument nach Anspruch 6 oder 7, **dadurch gekennzeichnet, dass** den distalseitigen freien Enden der Rohre (5, 7) im Übergang zu den vorstehenden Elektrodenspitzen (3, 4) den Außendurchmesser der Rohre (5, 7) reduzierende, nach schräg innen verlaufende Anschliffflächen (5a, 7a) ausgebildet sind.

9. Bipolares Koagulationsinstrument nach Anspruch 1, **dadurch gekennzeichnet, dass** die Drosselöffnung (11) im Bereich einer am proximalen Ende des Schaftes (1) angeordneten Handhabe (2) ausgebildet ist.

10. Bipolares Koagulationsinstrument nach Anspruch 1, **dadurch gekennzeichnet, dass** auch in der auf dem äußeren Rohr (7) angeordneten isolierenden Schicht (8) eine die Drosselöffnung (11) allseitig mit Abstand umgebende Aussparung (13) ausgebildet ist.

## Claims

1. Bipolar coagulation instrument with a hollow shaft (1) formed as a suction and/or rinsing tube and with two mutually electrically insulated electrodes (3, 4) which stick out beyond the distal end of the shaft (1), wherein the hollow shaft (1) consists of two mutually coaxially arranged, at least partially electrically conductive, tubes (5, 7) which are mutually insulated and insulated from the outside, wherein the tubes (5, 7) form the electrodes (3, 4),
**characterized in that**,
in the shaft (1), a throttle opening (11) is formed, wherein, in the area of the throttle opening (11), in the outer tube (7), a recess (12) enclosing the throttle opening (11) on all sides with spacing is formed.

2. Bipolar coagulation instrument according to Claim 1, **characterized in that** the tubes (5, 7) are mutually electrically insulated and electrically insulated from the outside by two layers (6, 8) which coaxially enclose the tubes (5, 7).

3. Bipolar coagulation instrument according to Claim 2, **characterized in that** the two tubes (5, 7) and the two insulating layers (6, 8) contact one another in a positivelocking manner.

4. Bipolar coagulation instrument according to Claim 2 or 3, **characterized in that** the insulating layers (6, 8) are formed as hoses, particularly shrunk hoses, applied on the inner tube (5) and the outer tube (7).

5. Bipolar coagulation instrument according to Claim 2 or 3, **characterized in that** the insulating layers (6, 8) are formed as coatings made of a plastic or ceramic material, applied on the inner tube (5) and on the outer tube (7).

6. Bipolar coagulation instrument according to one of Claims 1-5, **characterized in that**, at the distal ends of the inner tube (5) and of the outer tube (7), tube sections (3, 4) protruding like fingers are formed as electrode tips, which stick out beyond the distal end of the shaft (1).

7. Bipolar coagulation instrument according to Claim 6, **characterized in that** the tube sections (3, 4) which protrude like fingers are arranged facing each other at the distal end of the shaft (1).

8. Bipolar coagulation instrument according to Claim 6 or 7, **characterized in that** the distal-side free ends of the tubes (5, 7) in the transition to the protruding electrode tips (3, 4), polished surfaces (5a, 7a) are formed, which reduce the outer diameter of the tubes (5, 7) and which extend obliquely inward.

9. Bipolar coagulation instrument according to Claim 1, **characterized in that** the throttle opening (11) is formed in the area of a handle (2) arranged at the proximal end of the shaft (1).

10. Bipolar coagulation instrument according to Claim 1, **characterized in that** a recess (13), which encloses the throttle opening (11) on all sides with spacing, is also formed in the insulating layer (8) arranged on the outer tube (7).

## Revendications

1. Instrument de coagulation bipolaire avec une tige creuse (1) agencée sous forme de tube d'aspiration et/ou de rinçage ainsi qu'avec deux électrodes (3, 4) électriquement isolées l'une par rapport à l'autre, qui dépassent de l'extrémité distale de la tige (1), où la tige creuse (1) consiste en deux tubes (5, 7) au moins partiellement électriquement conducteurs, agencés coaxialement l'un sur l'autre, qui sont électriquement isolés l'un par rapport à l'autre et à l'extérieur, où les tubes (5, 7) forment les électrodes (3, 4),
**caractérisé en ce que**
une ouverture d'étranglement (11) est agencée dans la tige (1), où un évidement (12) entourant l'ouverture d'étranglement (11) de tous les côtés, à une certaine distance, est agencé dans le domaine de l'ouverture d'étranglement (11) dans le tube externe (7).

2. Instrument de coagulation bipolaire selon la revendication 1, **caractérisé en ce que** les tubes (5, 7) sont isolés électriquement l'un par rapport à l'autre et à l'extérieur par le biais de deux couches (6, 8) entourant coaxialement les tubes (5, 7).

3. Instrument de coagulation bipolaire selon la revendication 2, **caractérisé en ce que** les deux tubes (5, 7) et les deux couches isolantes (6, 8) sont disposés de manière contiguë par assemblage de forme.

4. Instrument de coagulation bipolaire selon la revendication 2 ou 3, **caractérisé en ce que** les couches isolantes (6, 8) sont agencées sous forme de tuyaux souples appliqués sur le tube interne (5) et le tube externe (7), en particulier sous forme de tuyaux souples rétractables.

5. Instrument de coagulation bipolaire selon la revendication 2 ou 3, **caractérisé en ce que** les couches isolantes (6, 8) sont agencées sous forme de revêtements appliqués sur le tube interne (5) et le tube externe (7) en un matériau synthétique ou céramique.

6. Instrument de coagulation bipolaire selon l'une des revendications 1 à 5, **caractérisé en ce que** des segments de tube (3, 4) en saillie à la manière de doigts, qui dépassent de l'extrémité distale de la tige (1), sont agencés au niveau des extrémités distales du tube interne (5) et du tube externe (7) sous forme de pointes d'électrodes.

7. Instrument de coagulation bipolaire selon la revendication 6, **caractérisé en ce que** les segments de tube (3, 4) en saillie à la manière de doigts sont agencés de manière mutuellement opposée à l'extrémité distale de la tige (1).

8. Instrument de coagulation bipolaire selon la revendication 6 ou 7, **caractérisé en ce qu'**au niveau des extrémités libres du côté distal des tubes (5, 7) dans la transition avec les pointes d'électrodes en saillie (3, 4) sont agencées des surfaces polies (5a, 7a) s'étendant en biais vers l'intérieur, réduisant le diamètre externe des tubes (5, 7).

9. Instrument de coagulation bipolaire selon la revendication 1, **caractérisé en ce que** l'ouverture d'étranglement (11) est agencée dans le domaine d'une manette (2) disposée à l'extrémité proximale de la tige (1).

10. Instrument de coagulation bipolaire selon la revendication 1, **caractérisé en ce qu'**un évidement (13) entourant de tous les côtés l'ouverture d'étranglement (11) à une certaine distance est agencé également dans la couche isolante (8) agencée sur le tube externe (7).
